# EUROPEAN PATENT SPECIFICATION

(11) **EP 2 246 698 B1**
(45) Date of publication and mention of the grant of the patent: **27.06.2012**
(21) Application number: 10160389.2
(22) Date of filing: 20.04.2010
(51) Int. Cl.: G01N 33/28

(54) **System and method for monitoring quality of fuels**
System und Verfahren zur Überwachung der Kraftstoffqualität
Système et procédé de surveillance de la qualité de carburants

(30) Priority: 30.04.2009 EP 09159157
(43) Date of publication of application: 03.11.2010
(73) Proprietor: Wärtsilä Schweiz AG, 8401 Winterthur (CH)
(72) Inventor: Belaid, Riad, 8052, Zürich (CH)
(74) Representative: Sulzer Management AG

(56) References cited:
- EP-A1- 1 715 323
- US-A- 5 982 847
- US-A1- 2004 213 373
- US-A1- 2006 156 820
- US-B1- 7 286 633
- RIEBEL ULRICH ET AL: "On-line measurement of particle size distribution and particle concentration in suspensions by ultrasonic spectrometry" CHEMICAL ENGINEERING AND TECHNOLOGY, WEINHEIM, DE, vol. 12, no. 6, 1 December 1989 (1989-12-01), pages 433-438, XP007912853 ISSN: 0930-7516
- VARDI J ET AL: "Improved test procedure for inorganic particles in fuel oil" ASTM SPECIAL TECHNICAL PUBLICATION, PHILADELPHIA, PA, US, 1 January 1985 (1985-01-01), pages 204-214, XP008113479 ISSN: 0066-0558

## Description

The invention relates to a system and a method for monitoring the quality of fuels supplied to a large diesel engine according to the preamble of claim 1 and claim 8 respectively and to a fuel supply for supplying fuel to a large diesel engine with such a system according to the preamble of claim 14.

There is an increasing interest in economic fuels for large diesel engines, especially for large diesel engines of the two-stroke type. Large diesel engines are for example used on ships, in power stations and in similar applications. The fuel most commonly used in such applications is Heavy Fuel Oil (HFO) and Marine Fuel Oil (MFO) having a density of 970 kg/m³ or more. HFO and MFO mostly consist of residues from the distillation of oil products or from cracking processes in refineries such as visbreaker or catalytic cracking processes, also called catcracker processes. Known catcracker processes make use of so-called "zeolites", i.e. synthetic alumino-silicates. Especially small particles of alumino-silcates of below 20 µm can reach the fractionating column and will thus be collected in the residue of the column. This residue forms part of most common HFO and MFO.

Known HFO and MFO are therefore contaminated with solid particles. These particles, mainly alumino-silicates or so-called "cat fines" are considered an epidemic and intricate issue in case they exceed the known specified limit of 15 ppm at the inlet of a large diesel engine. Insufficient treatment of the fuel oil can lead to higher concentrations of cat fines in the fuel and, thus, can cause harmful effects on the engine running behaviour such as accelerated wear ratio on several components, e. g. cylinder liner, piston rings, injection control units, etc.

Thus, it is essential that the HFO and MFO as bunkered must be treated before it enters the engine. Practical experience has shown that with proper treatment of HFO and MFO in a fuel separator such as a centrifugal separator a sum of aluminium and silicon of 80 ppm (80 mg/kg) can be reduced to less than 15 ppm which is usually considered as just tolerable. The most harmful cat fines are of the size of 10 µm to 20 µm. The fuel separators should therefore be capable of removing practically all particles larger than 10 µm. The fuel finally reaching the engine should not contain more than 15 ppm of aluminium and silicon.

Other contaminations like vanadium or sodium which lead to formation of deposits should not exceed the specified limits of 100 ppm and 30 ppm respectively as well.

A fuel analysis system and method for detecting cat fines by x-ray fluorescence is described in US 7 286 633.

It happens sometimes that one or more components of the fuel treatment system, e.g. an automatic filtering unit or a centrifugal separator, are improperly functioning and that the quality of the fuel is thus lowered thereby enabling an unacceptable amount of particles to enter the large diesel engine.

It is an object of the present invention to provide a system and a method for monitoring the quality of fuels supplied to a large diesel engine as well as a fuel supply for supplying fuel to a large diesel engine with such a system, which have the ability to detect and measure solid particles in HFO and MFO before engine inlet in real time, with solid particle concentrations down to 15 ppm or lower and with solid particle sizes down to 10 µm or smaller.

This object is satisfied in accordance with the invention by the system defined in claim 1, the method defined in claim 8 and the fuel supply defined in claim 14.

The system according to the invention for monitoring the quality of fuels supplied to a large diesel engine includes a supply tube portion for supplying fuel to the large diesel engine. The system in addition includes at least one source being an ultrasonic transmitter for emitting ultrasonic waves or a X-ray source for emitting X-rays respectively into fuel contained in the supply tube portion, at least one detector for producing a signal indicating the presence of solid particles in the fuel, with the detector being an ultrasonic receiver for detecting ultrasonic waves or a X-ray detector for detecting X-rays emitted respectively into the fuel contained in the supply tube portion, and a particle control unit connected to the detector or detectors for evaluating the signal with respect to the number and/or to the size of the solid particles in the fuel. The supply tube portion can for example have a square or rectangular cross section or at least two parallel sides facing each other. Optionally the source or sources can also be connected to the particle control unit. This e.g. allows determining time delay when ultrasonic transmitters are used.

In an advantageous embodiment variant the at least one source and/or the at least one detector are arranged on the same side of the supply tube portion for detecting solid particles in reflective mode, also called back scattering mode. Thus, the at least one detector can be arranged beside the at least one source on the same side of the supply tube portion or, in case a ultrasonic transducer is used, the transducer can be used both as a ultrasonic transmitter and as a ultrasonic receiver e.g. by providing the transducer with separate terminals for the transmitter and the receiver function or by switching the terminals of the transducer alternatively between a frequency generator used for transmitting ultrasonic waves and an amplifier used for receiving ultrasonic waves.

In a further advantageous embodiment variant the at least one source and the at least one detector are arranged on opposite sides of the supply tube portion respectively for detecting solid particles in a transmissive or absorptive mode.

According to the invention, the one or more sources are arranged so that they essentially irradiate an entire cross section of the supply tube portion. In a typical embodiment variant one or more sources and one or more detectors essentially extend across an entire side of a cross section of the supply tube portion.

The system can include two groups of sources, wherein the sources of the first group are located on one side of a cross section of the supply tube portion and the sources of the second group are located on another, in particular on an adjacent, side of the cross section. Alternatively or in addition the system can include two groups of detectors, wherein the detectors of the first group are located on one side of a cross section of the supply tube portion and the detectors of the second group are located on another, in particular on an adjacent, side of the cross section.

In a further embodiment variant the source or sources and/or the detector or detectors are arranged inside the supply tube portion or in or on at least one wall of the supply tube portion with an optional window or cover between the fuel in the supply tube portion and the sources or detectors.

The detector or detectors are advantageously capable of detecting solid particles with a diameter of less than 50 µm or less than 20 µm in the fuel. In a further embodiment variant the detector or detectors are capable of detecting solid particles with a diameter as small as 5 µm or as small as 2 µm or smaller and at concentrations down to 15 ppm or lower in the fuel. Typical detector or detectors are capable of detecting solid particles in the range between 5 µm to 50 µm.

In case ultrasonic transmitters and receivers are used the frequency range covered is preferably between 20 kHz and 100 MHz. Several ultrasonic transmitters and receivers are usually necessary because it may not be possible to cover the desired frequency range such as the frequency range between 20 kHz and 100 MHz with a single transmitter and a single receiver or likewise with a single transducer. Continuous or pulsed mode can be used for producing a signal indicating the presence of solid particles in the fuel. The frequency is advantageously swept within the desired frequency range.

The method according to the invention for monitoring the quality of fuels includes supplying fuel through a supply tubing to a large diesel engine. In addition the method includes emitting ultrasonic waves or X-rays into fuel contained in a supply tube portion by at least one source being an ultrasonic transmitter or a X-ray source respectively, detecting ultrasonic waves or X-rays emitted respectively into the fuel contained in the supply tube portion by at least one detector being an ultrasonic receiver for detecting ultrasonic waves or a X-ray detector, producing a signal indicating the presence of solid particles in the fuel by the at least one detector, and evaluating the signal with respect to the number and/or to the size of the solid particles in the fuel by a particle control unit connected to the detector or detectors. Optionally the source or sources can also be connected to the particle control unit. This e.g. allows determining time delay when ultrasonic transmitters are used.

The solid particles can be detected in a reflective mode, also called back scattering mode, in that the at least one source and/or the at least one detector are arranged on the same side of the supply tube portion. Thus, the at least one detector can be arranged beside the at least one source on the same side of the supply tube portion or, in case a ultrasonic transducer is used, the transducer can be used both as a ultrasonic transmitter and as a ultrasonic receiver e.g. by providing the transducer with separate terminals for the transmitter and the receiver function or by switching the terminals of the transducer alternatively between a frequency generator used for transmitting ultrasonic waves and an amplifier used for receiving ultrasonic waves.

The solid particles can further be detected in a transmissive or absorptive mode in that the at least one source and the at least one detector are arranged on opposite sides of the supply tube portion respectively.

The one or more sources essentially irradiate an entire cross section of the supply tube portion and typically one or more detectors are detecting ultrasonic waves or X-rays essentially from the entire cross section of the supply tube portion.

In an advantageous embodiment variant of the method fuel contained in a cross section of the supply tube portion is irradiated by two groups of sources, each group having an irradiation direction, wherein the irradiation direction of the first group of sources is different from the irradiation direction of the second group.

In a further advantageous embodiment variant of the method ultrasonic waves or X-rays are detected by two groups of detectors, each group detecting ultrasonic waves or X-rays having a specific direction, wherein the direction of the ultrasonic waves or X-rays detected by the first group of detectors is different from the direction of the ultrasonic waves or X-rays detected by the second group.

The angle between the irradiation direction of the first group of sources and the irradiation direction of the second group of sources or the angle between the direction of the ultrasonic waves or X-rays detected by the first group of detectors and the direction of the ultrasonic waves or X-rays detected by the second group of detectors can be between 60° and 120° or between 80° and 110° or essentially equal to 90°.

The method advantageously includes detecting solid particles with a diameter of less than 50 µm or less than 20 µm in the fuel. In a further advantageous embodiment variant the method includes detecting solid particles with a diameter as small as 5 µm or as small as 2 µm or smaller and at concentrations down to 15 ppm or lower in the fuel. The particle range detected is typically between 5 µm and 50 µm.

The main physical phenomena are considered a scattering effect which occurs when an ultrasonic wave hits a particle or a diffraction effect which occurs when X-rays hit a particle. In case of an ultrasonic wave the scattering affects the velocity and the attenuation of the ultrasonic wave in function of the frequency and in function of the distance, the incident angle and the characteristic of the particle or particles. An estimate for the concentration and size distribution of the particles can be determined by measuring the velocity and/or the time and/or the attenuation of the original signal in function of the frequency and by applying a mathematical model based for example on Fast Fourier Transformation. In case of X-rays the diffraction generates a diffraction pattern and affects the attenuation of the X-rays. The concentration and size distribution of the particles can e.g. be determined by measuring the attenuation which yields a direct image of the particles.

The invention further includes a fuel supply for supplying fuels to a large diesel engine, with the fuel supply including a fuel tank, a fuel supply tubing connected to the fuel tank and optionally a large diesel engine to which the fuel supply tubing is connected and/or a fuel treatment arrangement for removing impurities from fuel supplied from the fuel tank. The fuel supply additionally includes a system according to one or several of the embodiments or embodiment variants described above for monitoring the quality of fuels supplied to a large diesel engine.

The system and method according to the invention and the fuel supply according to the invention have the advantage that they enable the constant on-line monitoring of fuel oil quality with respect to solid particles in real time and that they thus allow taking appropriate counter measures in time such as servicing of the fuel treatment arrangement and/or, if possible, changing to an alternative fuel supply or activating an alternative fuel filter or separator. The system and method according to the invention and the fuel supply according to the invention have the further advantage that solid particles in the whole cross section of a supply tube portion can be detected. When more than one detector is used the signals from the detectors can be summed up in order to more clearly discriminate signals from background noise. Moreover, it is possible to determine the velocity of the fuel in a supply tube portion by determining the displacement of solid particles in a given time interval and/or to determine an estimate for the concentration and size distribution of the particles over a certain length of the supply tube portion, i.e. in a given volume of for example one liter or half a liter. It is further advantageous that all solid particles flowing through the supply tube portion can be detected even if the processing speed of the detectors, multiplexers and particle control unit is limited. When ultrasonic waves are used for producing a signal indicating the presence of solid particles in the fuel multiple transmitters and/or receivers and/or transducers can be provided covering different frequency ranges in order to detect particles of different sizes.

In the following the invention will be explained in more detail with reference to the specific embodiment and with reference to the drawing.
- Fig. 1: is a diagram of an exemplary embodiment of a fuel supply according to the present invention;
- Fig. 2: is a schematic view of an embodiment of a system for monitoring the quality of fuels according to the present invention;
- Fig. 3: is a detailed view of a detector arrangement of the embodiment shown in Fig. 2;
- Fig. 3A: is a detailed view of a detector arrangement of an embodiment variant;
- Fig. 4: is a cross section through a detector arrangement according to an embodiment variant of the present invention; and
- Fig. 5: is a cross section through a detector arrangement according to a further embodiment variant of the present invention.

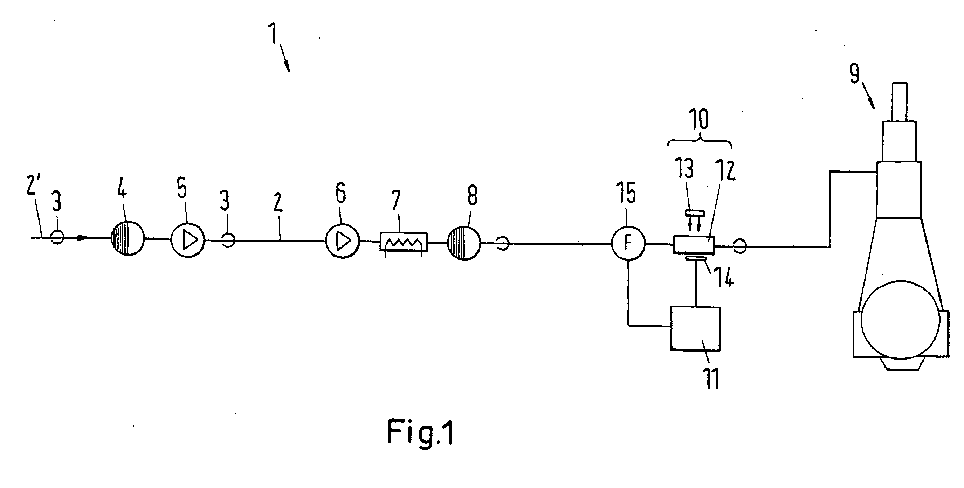

Fig. 1 shows a diagram of an exemplary embodiment of a fuel supply according to the present invention. In the embodiment the fuel supply 1 includes a fuel supply tubing 2, 2' and optionally one or more of the following components: one or more fuel tanks not shown in Fig. 1, a fuel treatment arrangement not shown in Fig. 1, a large diesel engine 9, a feed pump 5, a suction filter 4, a booster pump 6, an end heater 7, a fuel filter 8, a flow meter 15, as well as additional components if required. The fuel supply tubing 2, 2' is typically connected on the one side to one or more large diesel engines and on the other side to one or more fuel tanks and/or to a fuel treatment arrangement with the fuel treatment arrangement including e.g. one or more centrifugal separators and/or one or more fuel filtering units. Moreover, the fuel supply tubing 2, 2' usually includes a heating and a thermal insulation 3 for lowering the viscosity of the HFO and MFO conveyed.

In addition the fuel supply 1 includes a system 10 according the present invention for monitoring the quality of fuels supplied to a large diesel engine 9. The system according to the invention includes a supply tube portion 12 for supplying fuel to the large diesel engine. The system 10 in addition includes at least one source 13 being an ultrasonic transmitter for emitting ultrasonic waves or a X-ray source for emitting X-rays respectively into fuel contained in the supply tube portion 12, at least one detector 14 for producing a signal indicating the presence of solid particles in the fuel, with the detector being an ultrasonic receiver for detecting ultrasonic waves or a X-ray detector for detecting X-rays emitted respectively into the fuel contained in the supply tube portion, and a particle control unit 11 connected to the detector or detectors for evaluating the signal with respect to the number and/or to the size of the solid particles in the fuel. Optionally the source or sources 13 can also be connected to the particle control unit 11. The supply tube portion 12 can for example have a square or rectangular cross section or at least two parallel sides facing each other with the cross section being typically roughly equal to the cross section of the adjacent fuel supply tubing 2 or slightly bigger or smaller. In addition, the system 10 can optionally include a flow meter 15 such as an ultrasonic Doppler flow meter connected e.g. to the particle control unit 11.

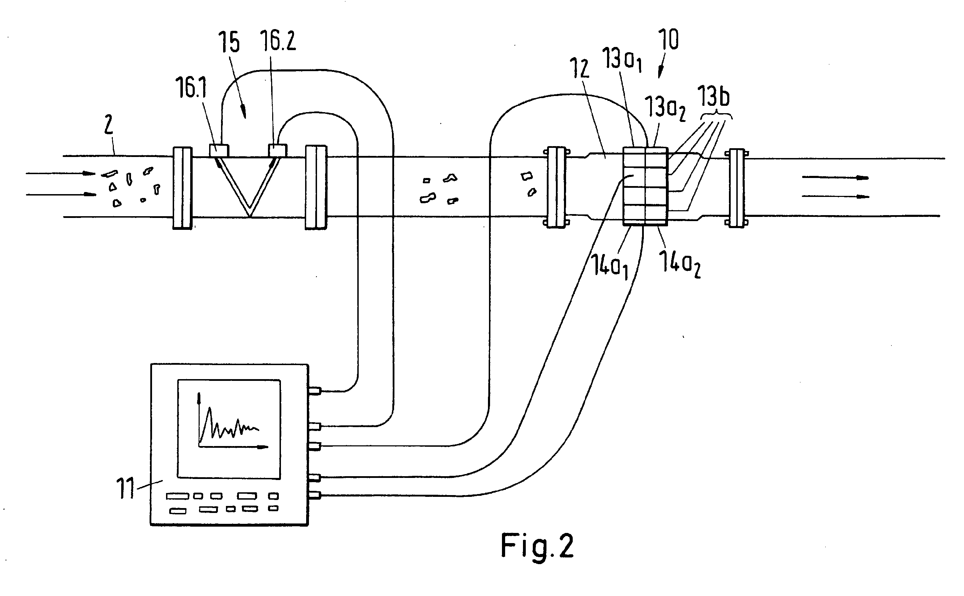

Fig. 2 shows a schematic view of a system 10 for monitoring the quality of fuels according to an embodiment of the present invention. The system 10 according to the embodiment shown in Fig. 2 includes a supply tube portion 12 for supplying fuel to a large diesel engine. The system 10 in addition includes at least one source 13a₁, 13a₂, 13b being an ultrasonic transmitter for emitting ultrasonic waves or a X-ray source for emitting X-rays respectively into fuel contained in the supply tube portion 12 and at least one detector 14a₁, 14a₂ for producing a signal indicating the presence of solid particles in the fuel, with the detector being an ultrasonic receiver for detecting ultrasonic waves or a X-ray detector for detecting X-rays emitted respectively into the fuel contained in the supply tube portion. The system 10 further includes a particle control unit 11 connected to the detector or detectors 14a₁, 14a₂ for evaluating the signal with respect to the number and/or to the size of the solid particles in the fuel. The supply tube portion 12 can for example have a square or rectangular cross section or at least two parallel sides facing each other. Optionally the source or sources 13a₁, 13a₂ can also be connected to the particle control unit 11. This e.g. allows determining time delay when ultrasonic transmitters are used.

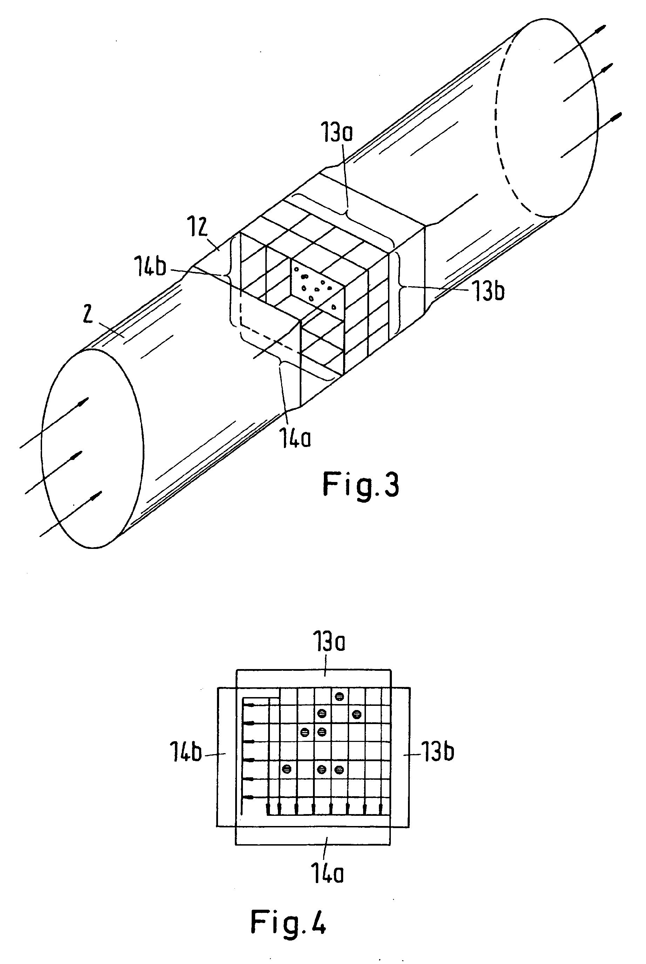

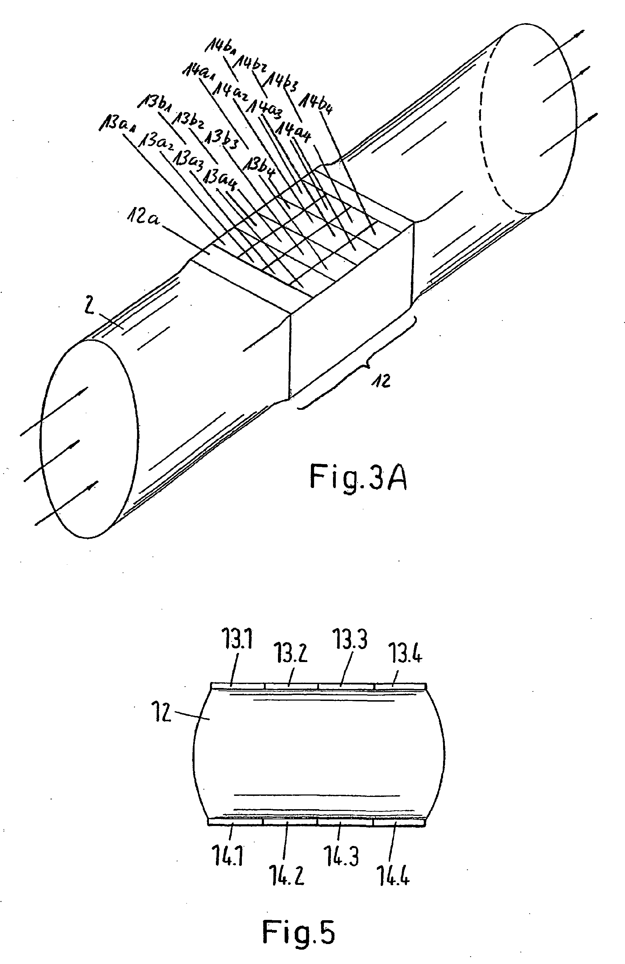

In an advantageous embodiment variant shown in Fig. 3A the at least one source 13a₁, 13a₂, 13b and/or the at least one detector 14a₁, 14a₂ are arranged on the same side of the supply tube portion 12 for detecting solid particles in reflective mode. In a further advantageous embodiment variant shown in Fig. 2 and 3 the at least one source 13a₁, 13a₂, 13b and the at least one detector 14a₁, 14a₂ are arranged on opposite sides of the supply tube portion 12 respectively for detecting solid particles in a transmissive or absorptive mode.

The source or sources 13a₁, 13a₂, 13b are arranged so that they essentially irradiate an entire cross section of the supply tube portion 12. In a typical embodiment variant the source or sources 13a₁, 13a_{2,} 13b and the detector or detectors 14a₁, 14a₂ essentially extend across an entire side of a cross section of the supply tube portion 12.

In a supply tube portion having a square cross section a side has typically a length of 10 cm to 15 cm. It can therefore be advantageous to use multiple sources and/or multiple detectors especially since the sources 13a₁, 13a₂, 13b and the one or more detectors 14a₁, 14a₂ essentially extend across an entire side of the cross section. The sources and/or the detectors can for example be arranged in a row of two, three, four or more pieces respectively. Moreover, it is possible to arrange more than one row on one side if required.

The system can as shown in Fig. 2 include two groups of sources 13aᵢ, 13b, wherein the sources 13aᵢ of the first group are located on one side of a cross section of the supply tube portion 12 and the sources 13b of the second group are located on another, in particular on an adjacent, side of the cross section. Alternatively or in addition the system can include two groups of detectors, wherein the detectors of the first group are located on one side of a cross section of the supply tube portion and the detectors of the second group are located on another, in particular on an adjacent, side of the cross section.

The detector or detectors 14a₁, 14a₂ are advantageously capable of detecting solid particles with a diameter of less than 50 µm or less than 20 µm in the fuel. In a further embodiment variant the detector or detectors are capable of detecting solid particles with a diameter as small as 5 µm or as small as 2 µm or smaller and at concentrations down to 15 ppm or lower in the fuel.

In an advantageous embodiment variant the detector or detectors 14a₁, 14a₂ are arranged inside the supply tube portion 12 or in or on a wall of the supply tube portion with an optional window or cover between the fuel in the supply tube portion and the detector or detectors.

If an X-ray source is used it is usually sufficient to provide one source at a distance of 0.1 m or more from the supply tube portion 12 in order to essentially irradiate an entire cross section of the supply tube portion. A typical X-ray source includes an X-ray tube which is an evacuated tube having a cathode and an anode. Electrons from the heated cathode are accelerated towards the anode and collide there with the anode target which typically contains tungsten, molybdenum, copper or a similar metal. Part of the energy of the electrons is emitted from the anode target as X-rays.

In an advantageous embodiment variant X-ray area detectors are used such as CCD chips or CMOS chips which are available up to a size of 2 inch by 3 inch (51 mm by 76 mm) or larger. CMOS chips can convert X-rays directly while CCD chips need to be preceded by a scintillator. The use of a scintillator has the advantage that the detector chips can be located out of the damaging X-ray beam by e.g. using optics such as fiber optics between the scintillator and the detector chips.

The system 10 can optionally include a flow meter 15 such as an ultrasonic Doppler flow meter connected e.g. to the particle control unit 11. In the embodiment shown in Fig. 2 the flow meter 15 includes two ultrasonic transceivers 16.1, 16. 2 arranged apart from each other on one side of a supply tube portion inserted into a supply tubing 2. One of the transceivers emits ultrasonic waves which are reflected on an opposite wall of the supply tube portion and the reflected waves are detected by the second transceiver. Thereby the frequency of the ultrasonic waves detected by the second transceiver depends on the velocity of the fuel flowing inside the supply tube portion.

In addition, the system 10 and/or the particle control unit 11 can include one or several of the following components not shown in Fig. 2.: a multiplexer or switching unit for switching between different sources 13a₁, 13a₂, 13b and/or detectors 14a₁, 14a₂; a frequency generator such as a pulse or a sweep generator for generating the signal or signals for the ultrasonic transmitters; an amplifier for amplifying the signals from the ultrasonic receivers; a digital to analog converter; or an analog to digital converter.

Fig. 3 shows a slanted view of a detector arrangement of the embodiment shown in Fig. 2 and described above. One or more detectors are arranged in or at a supply tube portion 12 which can for example have a square or rectangular cross section. In the embodiment variant shown in Fig. 3 at least one source 13a, 13b and at least one detector 14a, 14b are arranged on opposite sides of the supply tube portion 12 respectively for detecting solid particles in a transmissive or absorptive mode.

The source or sources 13a, 13b are arranged so that they essentially irradiate an entire cross section of the supply tube portion 12. In a typical embodiment variant the source or sources 13a, 13b and the detector or detectors 14a, 14b essentially extend across an entire side of a cross section of the supply tube portion 12.

In the embodiment shown in Fig. 3 a side of a cross section of the supply tube portion 12 has typically a length of 8 cm to 15 cm. It can therefore be advantageous to use multiple sources and/or multiple detectors especially since the sources 13a, 13b and the detectors 14a, 14b essentially extend across an entire side of the cross section. The sources and/or the detectors can for example be arranged in a row of two, three, four or more pieces respectively. Moreover, it is possible to arrange more than one row on one side if required.

The arrangement shown in Fig. 3 includes two groups of sources 13a, 13b, wherein the sources 13a of the first group are located on one side of a cross section of the supply tube portion 12 and the sources 13b of the second group are located on another, in particular on an adjacent, side of the cross section. Alternatively or in addition the system can include two groups of detectors 14a, 14b, wherein the detectors 14a of the first group are located on one side of a cross section of the supply tube portion and the detectors 14b of the second group are located on another, in particular on an adjacent, side of the cross section.

In an advantageous embodiment variant the detector or detectors 14a, 14b are arranged inside the supply tube portion 12 or in or on a wall of the supply tube portion with one or more optional windows or covers between the fuel in the supply tube portion and the detector or detectors.

Fig. 3A shows a slanted view of a source and detector arrangement of an embodiment variant. One or more sources 13a₁ - 13a₄, 13b₁ - 13b₄ and one or more detectors 14a₁ - 14a₄, 14b₁ - 14b₄ are arranged on one side 12a of a supply tube portion 12 for detecting solid particles in a reflective or back scattering mode. The supply tube portion 12 can for example have a square or rectangular cross section.

The source or sources 13a₁ - 13a_{4,} 13b₁ - 13b₄ are arranged so that they essentially irradiate an entire cross section of the supply tube portion 12. In a typical embodiment variant the source or sources 13a₁ - 13a₄, 13b₁ - 13b₄ and the detector or detectors 14a₁ - 14a₄, 14b₁ - 14b₄ essentially extend across the entire width of the side 12a.

In the embodiment shown in Fig. 3A the side 12a has typically a width of 8 cm to 15 cm. It can therefore be advantageous to use multiple sources and/or multiple detectors especially since the sources 13a₁ - 13a₄, 13b₁ - 13b₄ and the detectors 14a₁ - 14a₄, 14b₁ - 14b₄ essentially extend across the entire side 12a. The sources and/or the detectors can for example be arranged in a row of two, three, four or more pieces respectively. Moreover, it is possible to arrange more than one row if required.

Fig. 4 shows a cross section through a detector arrangement according to an embodiment variant of the present invention. One or more detectors 14a, 14b are arranged in or at a supply tube portion which can have a square or rectangular cross section. In the embodiment variant shown in Fig. 4 at least one source 13a, 13b and at least one detector 14a, 14b are arranged on opposite sides of the supply tube portion respectively for detecting solid particles in a transmissive or absorptive mode.

The source or sources 13a, 13b are arranged so that they essentially irradiate an entire cross section of the supply tube portion 12. In a typical embodiment variant the source or sources 13a, 13b and the detector or detectors 14a, 14b essentially extend across an entire side of a cross section of the supply tube portion. When an X-ray source or X-ray sources are used it can be advantageous to arrange the source or sources 13a, 13b at a distance from the supply tube portion.

In the embodiment shown in Fig. 4 a side of a cross section of the supply tube portion 12 has typically a length of 8 cm to 15 cm. It can therefore be advantageous to use multiple sources and/or multiple detectors especially since the sources 13a, 13b and the detectors 14a, 14b essentially extend across an entire side of the cross section. The sources and/or the detectors can for example be arranged in a row of two, three, four or more pieces respectively.

The arrangement shown in Fig. 4 includes two groups of sources 13a, 13b, wherein the sources 13a of the first group are located on one side of a cross section of the supply tube portion 12 and the sources 13b of the second group are located on another, in particular on an adjacent, side of the cross section. Alternatively or in addition the system can include two groups of detectors 14a, 14b, wherein the detectors 14a of the first group are located on one side of a cross section of the supply tube portion and the detectors 14b of the second group are located on another, in particular on an adjacent, side of the cross section.

In an advantageous embodiment variant the detector or detectors 14a, 14b are arranged inside the supply tube portion or in or on a wall of the supply tube portion with one or more optional windows or covers between the fuel in the supply tube portion and the detector or detectors.

Fig. 5 shows a cross section through a detector arrangement according to a further embodiment variant of the present invention. One or more detectors 14.1 - 14.4 are arranged in or at a supply tube portion 12 which has a cross section with two parallel sides facing each other. In the embodiment variant shown in Fig. 5 at least one source 13.1 -13.4 and at least one detector 14.1 - 14.4 are arranged on opposite sides of the supply tube portion respectively for detecting solid particles in a transmissive or absorptive mode.

The source or sources 13.1 -13.4 are arranged so that they essentially irradiate an entire cross section of the supply tube portion 12. In a typical embodiment variant the source or sources 13.1 -13.4 and the detector or detectors 14.1 - 14.4 essentially extend across an entire side of the cross section of the supply tube portion. When an X-ray source or X-ray sources are used it can be advantageous to arrange the source or sources at a distance from the supply tube portion.

In the embodiment shown in Fig. 5 a side of a cross section of the supply tube portion 12 has typically a length of 8 cm to 15 cm. It can therefore be advantageous to use multiple sources and/or multiple detectors especially since the sources 13.1 -13.4 and the detectors 14.1 - 14.4 essentially extend across an entire side of the cross section. The sources and/or the detectors can for example be arranged in a row of two, three, four or more pieces respectively.

In an advantageous embodiment variant the detector or detectors 14.1 - 14.4 are arranged inside the supply tube portion or in or on a wall of the supply tube portion with an optional window or cover between the fuel in the supply tube portion and the detector or detectors.

An embodiment of the method in accordance with the invention for monitoring the quality of fuels supplied to a large diesel engine will be described in the following with reference to Figures 1 to 5. The method includes supplying fuel through a supply tubing 2, 2' to a large diesel engine 9. In addition the method includes emitting ultrasonic waves or X-rays into fuel contained in a supply tube portion 12 by at least one source 13, 13a, 13b being an ultrasonic transmitter or a X-ray source respectively, detecting ultrasonic waves or X-rays emitted respectively into the fuel contained in the supply tube portion 12 by at least one detector 14, 14a, 14b being an ultrasonic receiver for detecting ultrasonic waves or a X-ray detector, producing a signal indicating the presence of solid particles in the fuel by the at least one detector, and evaluating the signal with respect to the number and/or to the size of the solid particles in the fuel by a particle control unit 11 connected to the detector or detectors 14, 14a, 14b. Optionally the source or sources 13, 13a, 13b can also be connected to the particle control unit 11.

The solid particles can be detected in a reflective mode in that the at least one source 13, 13a, 13b and/or the at least one detector 14, 14a, 14b are arranged on the same side of the supply tube portion 12 or they can be detected in a transmissive or absorptive mode in that the at least one source 13, 13a, 13b and the at least one detector 14, 14a, 14b are arranged on opposite sides of the supply tube portion 12 respectively.

The source or sources 13, 13a, 13b essentially irradiate an entire cross section of the supply tube portion 12 and typically the detector or detectors 14, 14a, 14b are detecting ultrasonic waves or X-rays essentially from the entire cross section of the supply tube portion.

Multiple sources can be used to transmit planar ultrasonic waves or planar microwaves and/or waves having a certain direction by controlling the phase of the waves transmitted by the individual sources. Moreover, multiple detectors can be used to receive ultrasonic waves or microwaves from a certain direction.

In an advantageous embodiment variant of the method fuel contained in a cross section of the supply tube portion 12 is irradiated by two groups of sources 13a, 13b, with each group having an irradiation direction, wherein the irradiation direction of the first group is different from the irradiation direction of the second group.

In a further advantageous embodiment variant of the method ultrasonic waves or X-rays are detected by two groups of detectors 14a, 14b, each group detecting ultrasonic waves or X-rays having a specific direction, wherein the direction of the ultrasonic waves or X-rays detected by the first group is different from the direction of the ultrasonic waves or X-rays detected by the second group.

The angle between the irradiation direction of the first group of sources 13a and the irradiation direction of the second group of sources 13b or the angle between the direction of the ultrasonic waves or X-rays detected by the first group of detectors 14a and the direction of the ultrasonic waves or X-rays detected by the second group of detectors 14b can be between 60° and 120° or between 80° and 110° or essentially equal to 90°.

The method advantageously includes detecting solid particles with a diameter of less than 50 µm or less than 20 µm in the fuel. In a further advantageous embodiment variant the method includes detecting solid particles with a diameter as small as 5 µm or as small as 2 µm or smaller and at concentrations down to 15 ppm or lower in the fuel.

The system and method according to the invention for monitoring the quality of fuels supplied to a large diesel engine as well as the fuel supply including such a system enable the constant on-line and in situ monitoring of fuel oil quality with respect to solid particles during operation of the diesel engine. Advantageously solid particles are monitored essentially in the whole cross section of a supply tube portion. Thus, it is possible to take counter measures in time if the particle count in the supplied fuel exceeds a specified limit. The system, method and fuel supply according to the invention therefore actively increases the service life of a large diesel engine.

## Claims

1. System for monitoring the quality of fuels supplied to a large diesel engine (9), including a supply tube portion (12) for supplying fuel to the large diesel engine, at least one source (13, 13a, 13b) being an ultrasonic transmitter for emitting ultrasonic waves or a X-ray source for emitting X-rays respectively into fuel contained in the supply tube portion (12) wherein the source or sources are able to essentially irradiate an entire cross section of the supply tube portion, at least one detector (14, 14a, 14b) for producing a signal indicating the presence of solid particles in the fuel, with the detector being an ultrasonic receiver for detecting scattered and/or attenuated ultrasonic waves or a X-ray detector for detecting diffracted or attenuated X-rays respectively, and a particle control unit (11) connected to the detector or detectors (14, 14a, 14b) for evaluating the signal with respect to the number and/or to the size of the solid particles in the fuel.

2. System according to claim 1, wherein the supply tube portion (12) has a square or rectangular cross section or at least two parallel sides facing each other.

3. System according to claim 1 or 2, wherein the at least one source (13, 13a, 13b) and/or the at least one detector (14, 14a, 14b) are arranged on the same side of the supply tube portion (12) for detecting solid particles in reflective mode, or wherein the at least one source (13, 13a, 13b) and the at least one detector (14, 14a, 14b) are arranged on opposite sides of the supply tube portion (12) respectively for detecting solid particles in a transmissive or absorptive mode.

4. System according to claim 3, wherein one or more detectors (14, 14a, 14b) essentially extend across an entire side of a cross section of the supply tube portion (12).

5. System according to one of the preceding claims including two groups of sources (13a, 13b), wherein the sources of the first group are located on one side of a cross section of the supply tube portion (12) and the sources of the second group are located on another, in particular on an adjacent, side of the cross section, and/or with the system including two groups of detectors (14a, 14b), wherein the detectors of the first group are located on one side of a cross section of the supply tube portion (12) and the detectors of the second group are located on another, in particular on an adjacent, side of the cross section.

6. System according to one of the preceding claims, wherein the source or sources 13, 13a, 13b and/or the detector or detectors 14, 14a, 14b are arranged inside the supply tube portion 12 or in or on at least one wall of the supply tube portion, in particular with a window or cover between the fuel in the supply tube portion and the sources or detectors.

7. System according to one of the preceding claims, wherein the detector or detectors (14, 14a, 14b) are capable of detecting solid particles with a diameter of less than 50 µm or less than 20 µm in the fuel, and/or wherein the detector or detectors (14, 14a, 14b) are capable of detecting solid particles with a diameter as small as 5 µm or as small as 2 µm or smaller and at concentrations down to 15 ppm or lower in the fuel.

8. Method for monitoring the quality of fuels, including supplying fuel through a supply tubing (2, 2') to a large diesel engine (9) **characterized in that** the method additionally includes emitting ultrasonic waves or X-rays into fuel contained in a supply tube portion (12) by at least one source (13, 13a, 13b) being an ultrasonic transmitter or a X-ray source respectively wherein the source or sources essentially irradiate an entire cross section of the supply tube portion, detecting scattered and/or attenuated ultrasonic waves or diffracted or attenuated X-rays respectively by at least one detector (14, 14a, 14b) being an ultrasonic receiver for detecting ultrasonic waves or a X-ray detector, producing a signal indicating the presence of solid particles in the fuel by the at least one detector (14, 14a, 14b), and evaluating the signal with respect to the number and/or to the size of the solid particles in the fuel by a particle control unit (11) connected to the detector or detectors (14, 14a, 14b).

9. Method according to claim 8, wherein the solid particles are detected in a reflective mode in that the at least one source (13, 13a, 13b) and/or the at least one detector (14, 14a, 14b) are arranged on the same side of the supply tube portion (12), or wherein the solid particles are detected in a transmissive or absorptive mode in that the at least one source (13, 13a, 13b) and the at least one detector (14, 14a, 14b) are arranged on opposite sides of the supply tube portion (12) respectively.

10. Method according to claim 8 or 9, wherein one or more detectors (14, 14a, 14b) are detecting ultrasonic waves or X-rays essentially from the entire cross section of the supply tube portion (12).

11. Method according to one of the claims 8 to 10, wherein the method includes irradiating fuel contained in a cross section of the supply tube portion (12) by two groups of sources (13a, 13b), with each group having an irradiation direction, and wherein the irradiation direction of the first group of sources is different from the irradiation direction of the second group of sources, and/or wherein the method includes detecting ultrasonic waves or X-rays by two groups of detectors (14a, 14b), with each group detecting ultrasonic waves or X-rays having a specific direction and wherein the direction of the ultrasonic waves or X-rays detected by the first group of detectors is different from the direction of the ultrasonic waves or X-rays detected by the second group of detectors.

12. Method according to claim 11, wherein the angle between the irradiation direction of the first group of sources and the irradiation direction of the second group of sources and/or the angle between the direction of the ultrasonic waves or X-rays detected by the first group of detectors and the direction of the ultrasonic waves or X-rays detected by the second group of detectors are between 60° and 120° or between 80° and 110° or essentially equal to 90°.

13. Method according to one of claims 8 to 12, wherein the method includes detecting solid particles with a diameter of less than 50 µm or less than 20 µm in the fuel, and/or wherein the method includes detecting solid particles with a diameter as small as 5 µm or as small as 2 µm or smaller and at concentrations down to 15 ppm or lower in the fuel.

14. Fuel supply (1) for supplying fuels to a large diesel engine (9), including a fuel tank and a fuel supply tubing (2, 2') connected to the fuel tank, wherein the fuel supply (1) additionally includes a system (10) in accordance with one of the claims 1 to 7 for monitoring the quality of fuels supplied to a large diesel engine.

15. Fuel supply according to claim 14 including additionally a large diesel engine (9) to which the fuel supply tubing (2, 2') is connected and/or a fuel treatment arrangement for removing impurities from fuel supplied from the fuel tank.

## Patentansprüche

1. System zum Überwachen der Qualität von Kraftstoffen, die an einen Groß-Dieselmotor (9) geliefert werden, mit einem Lieferrohrabschnitt (12) zum Liefern von Kraftstoff an den Groß-Dieselmotor, zumindest einer Quelle (13, 13a, 13b), die ein Ultraschallsender zum Aussenden von Ultraschallwellen bzw. eine Röntgenquelle zum Aussenden von Röntgenstrahlen in Kraftstoff, der in dem Lieferrohrabschnitt (12) enthalten ist, ist, wobei die Quelle oder die Quellen in der Lage sind, einen gesamten Querschnitt des Lieferrohrabschnittes im Wesentlichen zu bestrahlen, zumindest einem Detektor (14, 14a, 14b) zur Erzeugung eines Signals, das die Anwesenheit von Feststoffpartikeln in dem Kraftstoff angibt, wobei der Detektor ein Ultraschallempfänger zum Detektieren gestreuter und/oder abgeschwächter Ultraschallwellen bzw. ein Röntgendetektor zur Detektion gebeugter oder abgeschwächter Röntgenstrahlen ist, und einer Partikelsteuereinheit (11), die mit dem Detektor oder den Detektoren (14, 14a, 14b) verbunden ist, zur Bewertung des Signals in Bezug auf die Anzahl und/oder die Größe der Feststoffpartikel in dem Kraftstoff.

2. System nach Anspruch 1,
wobei der Lieferrohrabschnitt (12) einen quadratischen oder rechtwinkligen Querschnitt oder zumindest zwei parallele Seiten, die zueinander weisen, besitzt.

3. System nach einem der Ansprüche 1 oder 2,
wobei die zumindest eine Quelle (13, 13a, 13b) und/oder der zumindest eine Detektor (14, 14a, 14b) auf derselben Seite des Lieferrohrabschnittes (12) zur Detektion von Feststoffpartikeln im reflektiven Modus angeordnet sind oder wobei die zumindest eine Quelle (13, 13a, 13b) und der zumindest eine Detektor (14, 14a, 14b) jeweils auf gegenüberliegenden Seiten des Lieferrohrabschnittes (12) zur Detektion von Feststoffpartikeln in einem transmissiven oder absorptiven Modus angeordnet sind.

4. System nach Anspruch 3,
wobei ein oder mehrere Detektoren (14, 14a, 14b) sich im Wesentlichen über eine gesamte Seite eines Querschnittes des Lieferrohrabschnittes (12) erstrecken.

5. System nach einem der vorhergehenden Ansprüche,
mit zwei Gruppen von Quellen (13a, 13b), wobei die Quellen der ersten Gruppe auf einer Seite eines Querschnitts des Lieferrohrabschnittes (12) angeordnet sind und die Quellen der zweiten Gruppe auf einer anderen, insbesondere auf einer benachbarten Seite des Querschnittes angeordnet sind und/oder wobei das System zwei Gruppen von Detektoren (14a, 14b) aufweist, wobei die Detektoren der ersten Gruppe auf einer Seite eines Querschnitts des Lieferrohrabschnittes (12) angeordnet sind und die Detektoren der zweiten Gruppe auf einer anderen, insbesondere auf einer benachbarten Seite des Querschnittes angeordnet sind.

6. System nach einem der vorhergehenden Ansprüche,
wobei die Quelle oder die Quellen (13, 13a, 13b) und/oder der Detektor oder die Detektoren (14, 14a, 14b) innerhalb des Lieferrohrabschnittes (12) oder in oder an zumindest einer Wand des Lieferrohrabschnittes, insbesondere mit einem Fenster oder einer Abdeckung zwischen dem Kraftstoff in dem Lieferrohrabschnitt und den Quellen oder Detektoren angeordnet sind.

7. System nach einem der vorhergehenden Ansprüche,
wobei der Detektor oder die Detektoren (14, 14a, 14b) in der Lage sind, Feststoffpartikel mit einem Durchmesser von kleiner als 50 µm oder kleiner als 20 µm in dem Kraftstoff zu detektieren, und/oder wobei der Detektor oder die Detektoren (14, 14a, 14b) in der Lage sind, Feststoffpartikel mit einem Durchmesser von so klein wie 5 µm oder so klein wie 2 µm oder kleiner und bei Konzentrationen hinunter auf 15 ppm oder geringer in dem Kraftstoff zu detektieren.

8. Verfahren zum Überwachen der Qualität von Kraftstoffen, mit einem Liefern von Kraftstoff durch eine Lieferverrohrung (2, 2') an einen Groß-Dieselmotor (9),
**dadurch gekennzeichnet, dass**
das Verfahren zusätzlich ein Aussenden von Ultraschallwellen oder Röntgenstrahlen in Kraftstoff, der in einem Lieferrohrabschnitt (12) enthalten ist, durch zumindest eine Quelle (13, 13a, 13b), die ein Ultraschallsender bzw. eine Röntgenquelle ist, wobei die Quelle oder die Quellen im Wesentlichen einen gesamten Querschnitt des Lieferrohrabschnittes bestrahlen, ein Detektieren gestreuter und/oder abgeschwächter Ultraschallwellen bzw. gebeugter oder abgeschwächter Röntgenstrahlen durch zumindest einen Detektor (14, 14a, 14b), der ein Ultraschallempfänger zur Detektion von Ultraschallwellen oder ein Röntgendetektor ist, ein Erzeugen eines Signals, das die Anwesenheit von Feststoffpartikeln in dem Kraftstoff angibt, durch den zumindest einen Detektor (14, 14a, 14b), und ein Bewerten des Signals in Bezug auf die Anzahl und/oder die Größe der Feststoffpartikel in dem Kraftstoff durch eine Partikelsteuereinheit (11) umfasst, die mit dem Detektor oder den Detektoren (14, 14a, 14b) verbunden ist.

9. Verfahren nach Anspruch 8,
wobei die Feststoffpartikel in einem reflektiven Modus detektiert werden, in dem die zumindest eine Quelle (13, 13a, 13b) und/oder der zumindest eine Detektor (14, 14a, 14b) auf derselben Seite des Lieferrohrabschnittes (12) angeordnet sind, oder wobei die Feststoffpartikel in einem transmissiven oder absorptiven Modus detektiert werden, in dem die zumindest eine Quelle (13, 13a, 13b) und der zumindest eine Detektor (14, 14a, 14b) jeweils auf gegenüberliegenden Seiten des Lieferrohrabschnittes (12) angeordnet sind.

10. Verfahren nach einem der Ansprüche 8 oder 9,
wobei ein oder mehrere Detektoren (14, 14a, 14b) Ultraschallwellen oder Röntgenstrahlen im Wesentlichen von dem gesamten Querschnitt des Lieferrohrabschnittes (12) detektieren.

11. Verfahren nach einem der Ansprüche 8 bis 10,
wobei das Verfahren ein Bestrahlen von Kraftstoff, der in einem Querschnitt des Lieferrohrabschnittes (12) enthalten ist, durch zwei Gruppen von Quellen (13a, 13b) umfasst, wobei jede Gruppe eine Bestrahlungsrichtung aufweist, und wobei die Bestrahlungsrichtung der ersten Gruppe von Quellen von der Bestrahlungsrichtung der zweiten Gruppe von Quellen verschieden ist, und/oder wobei das Verfahren ein Detektieren von Ultraschallwellen oder Röntgenstrahlen durch zwei Gruppen von Detektoren (14a, 14b) umfasst, wobei jede Gruppe Ultraschallwellen oder Röntgenstrahlen mit einer spezifischen Richtung detektiert, und wobei die Richtung der Ultraschallwellen oder Röntgenstrahlen, die durch die erste Gruppe von Detektoren detektiert werden, sich von der Richtung der Ultraschallwellen oder Röntgenstrahlen unterscheidet, die durch die zweite Gruppe von Detektoren detektiert werden.

12. Verfahren nach Anspruch 11,
wobei der Winkel zwischen der Bestrahlungsrichtung der ersten Gruppe von Quellen und der Bestrahlungsrichtung der zweiten Gruppe von Quellen und/oder der Winkel zwischen der Richtung der Ultraschallwellen oder Röntgenstrahlen, die durch die erste Gruppe von Detektoren detektiert werden, und der Richtung der Ultraschallwellen oder Röntgenstrahlen, die durch die zweite Gruppe von Detektoren detektiert werden, zwischen 60° und 120° oder zwischen 80° und 110° oder im Wesentlichen bei gleich 90° liegen.

13. Verfahren nach einem der Ansprüche 8 bis 12,
wobei das Verfahren das Detektieren von Feststoffpartikeln mit einem Durchmesser von weniger als 50 µm oder weniger als 20 µm in dem Kraftstoff umfasst und/oder wobei das Verfahren ein Detektieren von Feststoffpartikeln mit einem Durchmesser von so klein wie 5 µm oder so klein wie 2 µm oder kleiner und bei Konzentrationen hinunter auf 15 ppm oder geringer in dem Kraftstoff umfasst.

14. Kraftstofflieferung (1) zum Liefern von Kraftstoffen an einen Groß-Dieselmotor (9), mit einem Kraftstofftank und einer Kraftstofflieferverrohrung (2, 2'), die mit dem Kraftstofftank verbunden ist, wobei die Kraftstofflieferung (1) zusätzlich ein System (10) gemäß einem der Ansprüche 1 bis 7 zur Überwachung der Qualität von Kraftstoffen, die an einen Groß-Dieselmotor geliefert werden, umfasst.

15. Kraftstofflieferung nach Anspruch 14,
zusätzlich mit einem Groß-Dieselmotor (9), mit dem die Kraftstofflieferverrohrung (2, 2') verbunden ist, und/oder einer Kraftstoffbehandlungsanordnung zum Entfernen von Unreinheiten von Kraftstoff, der von dem Kraftstofftank geliefert wird.

## Revendications

1. Système de surveillance de la qualité de combustibles fournis à un grand moteur Diesel (9), comprenant une portion de tube d'amenée (12) pour amener le combustible au grand moteur Diesel, au moins une source (13, 13a, 13b) étant un transmetteur ultrasonique pour émettre des ondes ultrasoniques ou bien une source de rayons X pour émettre des rayons X respectivement dans le combustible se trouvant dans la portion de tube d'amenée (12), où la ou les sources sont aptes à irradier essentiellement toute une section transversale de la portion de tube d'amenée, au moins un détecteur (14, 14a, 14b) pour produire un signal indiquant la présence de particules solides dans le combustible, le détecteur étant un récepteur ultrasonique pour détecter des ondes ultrasoniques dispersées et/ou atténuées ou bien un détecteur de rayons X pour détecter des rayons X diffractés ou atténués, respectivement, et une unité de commande de particules (11) reliée au ou aux détecteurs (14, 14a, 14b) pour évaluer le signal relativement au nombre et/ou à la taille des particules solides dans le combustible.

2. Système selon la revendication 1, dans lequel la portion de tube d'amenée (12) a une section transversale carrée ou rectangulaire ou au moins deux côtés parallèles se faisant face.

3. Système selon la revendication 1 ou 2, dans lequel la au moins une source (13, 13a, 13b) et/ou le au moins un détecteur (14, 14a, 14b) sont agencés sur le même côté de la portion de tube d'amenée (12) pour détecter des particules solides en un mode de réflexion, ou bien où la au moins une source (13, 13a, 13b) et le au moins un détecteur (14, 14a, 14b) sont agencés sur des côtés opposés de la portion de tube d'amenée (12) respectivement pour détecter des particules solides en un mode de transmission ou d'absorption.

4. Système selon la revendication 3, dans lequel un ou plusieurs détecteurs (14, 14a, 14b) s'étendent essentiellement sur tout un côté de la section transversale de la portion de tube d'amenée (12).

5. Système selon l'une des revendications précédentes, comprenant deux groupes de sources (13a, 13b) où les sources du premier groupe se situent sur un côté d'une section transversale de la portion de tube d'amenée (12), et les sources du deuxième groupe se situent sur un autre, en particulier sur un côté adjacent de la section transversale, et/ou le système incluant deux groupes de détecteurs (14a, 14b), où les détecteurs du premier groupe se situent sur un côté d'une section transversale de la portion de tube d'amenée (12), et les détecteurs du deuxième groupe se situent sur un autre, en particulier sur un côté adjacent de la section transversale.

6. Système selon l'une des revendications précédentes, dans lequel la ou les sources (13, 13a, 13b) et/ou le ou les détecteurs (14, 14a, 14b) sont agencés à l'intérieur de la portion de tube d'amenée (12) ou dans ou sur au moins une paroi de la portion de tube d'amenée, en particulier avec une fenêtre ou couvercle entre le combustible dans la portion de tube d'amenée et les sources ou détecteurs.

7. Système selon l'une des revendications précédentes, dans lequel le ou les détecteurs (14, 14a, 14b) sont aptes à détecter des particules solides d'un diamètre inférieur à 50 µm ou inférieur à 20 µm dans le combustible, et/ou où le ou les détecteurs (14, 14a, 14b) sont aptes à détecter des particules solides d'un diamètre aussi petit que 5 µm ou aussi petit que 2µm ou plus petit et à des concentrations jusqu'à 15 ppm ou inférieures dans le combustible.

8. Procédé de surveillance de la qualité de combustibles incluant l'amenée du combustible à travers un tube d'amenée (2, 2') à un grand moteur Diesel (9), **caractérisé en ce que** le procédé comprend additionnellement l'émission d'ondes ultrasoniques ou de rayons X dans le combustible se trouvant dans une portion de tube d'amenée (12) par au moins une source (13, 13a, 13b) qui est un transmetteur ultrasonique ou une source de rayons X respectivement, où la ou les sources irradient essentiellement toute une section transversale de la portion de tube d'amenée, en détectant des ondes ultrasoniques dispersées et/ou atténuées ou des rayons X diffractés ou atténués respectivement par au moins un détecteur (14, 14a, 14b) qui est un récepteur ultrasonique pour détecter des ondes ultrasoniques ou un détecteur de rayons X, produisant un signal indiquant la présence de particules solides dans le combustible par le au moins un détecteur (14, 14a, 14b), et évaluer le signal relativement au nombre et/ou à la taille des particules solides dans le combustible par une unité de commande de particules (11) connectée au ou aux détecteurs (14, 14a, 14b).

9. Procédé selon la revendication 8, dans lequel les particules solides sont détectées en un mode de réflexion en ce que la au moins une source (13, 13a, 13b) et/ou le au moins un détecteur (14, 14a, 14b) sont agencés sur le même côté de la portion de tube d'amenée (12), ou bien où les particules solides sont détectées en un mode de transmission ou d'absorption en ce que la au moins une source (13, 13a, 13b) et le au moins un détecteur (14, 14a, 14b) sont agencés sur des côtés opposés de la portion de tube d'amenée (12), respectivement.

10. Procédé selon la revendication 8 ou 9, dans lequel un ou plusieurs détecteurs (14, 14a, 14b) détectent des ondes ultrasoniques ou des rayons X essentiellement de toute la section transversale de la portion de tube d'amenée (12).

11. Procédé selon l'une des revendications 8 à 10, dans lequel le procédé comprend l'irradiation du combustible contenu dans une section transversale de la portion de tube d'amenée (12) par deux groupes de sources (13a, 13b), chaque groupe ayant une direction d'irradiation, et où la direction d'irradiation du premier groupe de sources diffère de la direction d'irradiation du deuxième groupe de sources, et/ou où le procédé comprend la détection d'ondes ultrasoniques ou de rayons X par deux groupes de détecteurs (14a, 14b), chaque groupe détectant des ondes ultrasoniques ou des rayons X ayant une direction spécifique, et où la direction des ondes ultrasoniques ou rayons X détectés par le premier groupe de détecteurs diffère de la direction des ondes ultrasoniques ou rayons X détectés par le deuxième groupe de détecteurs.

12. Procédé selon la revendication 11, dans lequel l'angle entre la direction d'irradiation du premier groupe de sources et la direction d'irradiation du deuxième groupe de sources et/ou l'angle entre la direction des ondes ultrasoniques ou rayons X détectés par le premier groupe de détecteurs et la direction des ondes ultrasoniques ou rayons X détectés par le deuxième groupe de détecteurs sont entre 60° et 120° et entre 80° et 110°, ou essentiellement égaux à 90°.

13. Procédé selon l'une des revendications 8 à 12, dans lequel le procédé comprend la détection des particules solides d'un diamètre inférieur à 50µm ou inférieur à 20µm dans le combustible, et/ou où le procédé comprend la détection de particules solides d'un diamètre aussi petit que 5µm ou aussi petit que 2µm ou plus petit et à des concentrations jusqu'à 15 ppm ou inférieures dans le combustible.

14. Amenée de combustible (1) pour amener des combustibles à un grand moteur Diesel (9), incluant un réservoir de combustible et un tube d'amenée de combustible (2, 2') relié au réservoir de combustible, où l'amenée de combustible (1) comprend additionnellement un système (10) en accord avec une des revendications 1 à 7 pour surveiller la qualité des combustibles fournis à un grand moteur Diesel.

15. Amenée de combustible selon la revendication 14, incluant additionnellement un grand moteur Diesel (9) auquel le tube d'amenée de combustible (2, 2') est relié et/ou un agencement de traitement du combustible pour supprimer les impuretés dans le combustible fourni par le réservoir de combustible.
